# EUROPEAN PATENT SPECIFICATION

(11) **EP 3 910 302 B1**
(45) Date of publication and mention of the grant of the patent: **12.08.2026**
(21) Application number: 19908296.7
(22) Date of filing: 26.12.2019
(51) Int. Cl.: A61B 5/01, A61B 5/00, G01J 5/00, G01J 5/02, G01J 5/04

(54) **EAR THERMOMETER WITH HOLDER**
OHRTHERMOMETER MIT HALTER
THERMOMÈTRE AURICULAIRE AVEC SUPPORT

(30) Priority: 10.01.2019 JP 2019002521
(43) Date of publication of application: 17.11.2021
(73) Proprietor: Bio Echo Net Inc., Sapporo-shi, Hokkaido 064-0804 (JP)
(72) Inventor: TANAKA Hideki, Sapporo-shi, Hokkaido 064-0804 (JP)
(74) Representative: Marks & Clerk LLP
(86) International application number: PCT/JP2019/051144
(87) International publication number: WO 2020/145179

(56) References cited:
- EP-A1- 3 214 850
- EP-A1- 3 312 578
- WO-A1-2016/036925
- WO-A1-2016/204048
- WO-A1-2017/019885
- WO-A1-2017/048476
- WO-A1-2017/203251
- WO-A2-2018/227125
- JP-A- 2006 203 420
- JP-A- H06 319 705
- KR-A- 20180 106 781
- US-A- 5 469 855
- US-A1- 2016 261 942

## Description

### TECHNICAL FIELD

The present invention relates to a holder for holding a thermometer in an ear for measuring a body temperature of a temperature measurement target person, and an ear thermometer using the holder.

### BACKGROUND

For example, in an operating room, an intensive care unit or the like, it is essential to measure a body temperature of a temperature measurement target person during the operation.

Further, for example, it may be necessary to measure a body temperature as part of physical condition management for workers who work for a long period of time with a large physical burden and athletes who play various sports.

Since it is necessary to continuously measure a body temperature of a temperature measurement target person such as a patient, a worker, or an athlete over a long period of time, it is important to reduce the burden on the body.

As a thermometer that meets such demands, an ear thermometer for measuring a temperature of an eardrum by inserting a probe into an ear hole of a temperature measurement target person has been proposed (see PTL 1).

EP3312578A1 generally relates to a thermometer including a probe to be fitted into an ear of a patient, the probe having an infrared sensor for measuring the temperature of an eardrum of the ear of the patient in a non-contact manner, a signal cable connected to the infrared sensor and drawn out of the probe, a gripping part provided in the probe to be gripped when the probe is inserted inside a tragus of the ear, and a groove portion provided in the gripping part to hold the signal cable in a curved state and to allow a curved portion of the signal cable to fit in and along a cavum conchae of the ear.

WO2017203251A1 generally relates to wearable devices capable of measuring a core body temperature and other vital signs of a user in a range of situations. The wearable device is arranged to be retained within the ear canal of the ear, in order to prevent the wearable device from inadvertently removing itself from the ear. Providing an infrared thermopile at the innermost end of the ear insert ensures that the infrared thermopile is provided as close as possible to the tympanic membrane which will be used to provide an indication of the core body temperature. The device has an audio conduction channel at least partly defined within an ear canal extending member, the audio conduction channel configured as a waveguide to conduct sound through a blocking member to a distal portion of the ear insert.

WO2017048476A1 generally relates to earbuds configured with one or more biometric sensors. At least one of the biometric sensors is configured to be pressed up against a portion of the tragus for making biometric measurements. In some embodiments, the housing of the earbud can be symmetric so that the earbud can be worn interchangeably in either a left or a right ear of a user. In such an embodiment, the earbud can include a sensor and circuitry configured to determine and alter operation of the earbud in accordance to which ear the earbud is determined to be positioned within.

WO2017019885A1 generally relates to an in-ear earphone including a body shaped to fit in the wearers ear, a nozzle extending from the body towards the ear canal of the wearers ear, the nozzle including an acoustic passage to conduct sound waves to the ear canal of the wearer, an ear canal sealing structure extending from the nozzle, and a sensor coupled to the nozzle. The sealing structure includes a thin sheet of material forming a hollow shape surrounding the nozzle and sensor, and the body, nozzle, sealing structure, and sensor are arranged such that when the earphone is located in the wearers ear, the sensor faces the tragus of the ear, and the sealing structure simultaneously forms an acoustic seal to the entrance to the ear canal and provides optical coupling between the sensor and the tragus with minimal air gaps between the sensor, the sealing structure, and the tragus.

US2016261942A1 generally relates to an earbud headphone adapter configured to securely retain an earbud headphone in a user's ear, preserve the quality of sound emitted from the earbud headphone into a user's ear, and/or reduce the discomfort commonly experienced by users from prolonged wearing of an earbud headphone. A hollow body having a cavity extending from a first plane to an earbud opening about an axis substantially perpendicular to a second plane is configured to stretch over and securely retain an earbud headphone. A portion of the hollow body may taper from the earbud opening to a sound opening. A stabilizer extends from the hollow body generally along the first plane and is configured to engage with the concha area of a user's ear while the sound opening is within the ear canal opening to securely retain the earbud adapter and earbud headphone in the user's ear.

KR20180106781A generally relates to providing a thermometer with improved wearability while minimizing the volume. The thermometer comprises: a main body having a first body including first and second regions and a second body mounted on the first body, and extended in a first direction; a rubber cap surrounding the first region and formed to be inserted into the ear; a temperature sensor disposed in the second region, and sensing the temperature having a specific temperature sensing range with respect to the first direction; and first and second circuit boards electrically connected to the temperature sensor, and disposed in the second region in a second direction intersecting with the first direction.

WO2016036925A1 generally relates to an earpiece that includes a body, a retaining structure, and a movable joint structure. The body includes an acoustic passage for conducting sound waves. The retaining structure is configured to engage with external structural features to hold the body in position, and the movable joint structure is configured to couple the body to the retaining structure. The movable joint structure is also configured to allow angular motion of the body with respect to the retaining structure.

JP2006203420A generally relates to providing a pinna support member and an earphone which enable wearing with a preferable wearing feeling without a slack nor a fall. The earphone includes at least a housing in which a speaker unit is incorporated and the pinna support member which is fitted to the housing to stop the earphone from falling from the pinna side. The pinna support member is composed of a fitting portion which is formed to freely be fitted to the housing and an elastic support piece portion which is projected from the fitting portion to freely come into elastic contact with a corresponding position in the pinna.

EP3214850A1 generally relates to a sound output device worn on an ear of a listener and used, and having listening characteristics of an ambient sound in a wearing state. A sound output device includes a sound generating portion that generates a sound, a sound guiding portion that takes in the sound generated in the sound generating portion from one end, and a holding portion that holds the sound guiding portion in the vicinity of the other end. The holding portion is engaged with an intertragic notch, and supports the sound guiding portion such that a sound output hole of the other end of the sound guiding portion to face a depth side of an ear canal. Even in a state where the listener wears the sound output device, the sound output device does not block an ear cavity of the listener, and the listener can listen to the ambient sound.

### CITATION LIST

### Patent Literature

PTL 1: JP 5039618 B2

### SUMMARY

Here, an ear thermometer measures a body temperature by detecting infrared rays emitted from an eardrum with an infrared sensor.

Therefore, in order to accurately measure a body temperature, it is necessary to hold an ear thermometer in an ear canal so that a measurement portion of the infrared sensor faces the eardrum side.

However, depending on the movement of a head of a temperature measurement target person, the position of an ear thermometer may shift in an ear canal, and accordingly the measurement portion of the infrared sensor may not accurately point to the eardrum.

In particular, when a worker who performs various tasks or an athlete who engages in various sports wears an ear thermometer, there is a risk that the position of the ear thermometer will be greatly displaced in an ear canal due to vigorous movement, and accordingly there is an inconvenience that an accurate body temperature measurement cannot be performed.

The present invention has been made in view of the above problems, and an object of the present invention is to provide a holder capable of stably holding an infrared sensor in a state where the infrared sensor can efficiently receive infrared rays emitted from an eardrum, and an ear thermometer using the holder.

The present invention provides an ear thermometer with a holder of claim 1 and a use of claim 3.

In order to achieve the above object, an ear thermometer with a holder according to a first aspect of the present invention is recited in claim 1.

As a result, the holder of the ear thermometer can stably hold the ear thermometer in a state where infrared rays emitted from an eardrum can be efficiently received by the infrared sensor.

A flexible joint portion is formed inside an intersection of the engaging arm and the support portion.

This makes it possible to improve the retentiveness of the ear thermometer in an ear by the holder of the ear thermometer.

A part of the engaging arm may be formed so as to be engageable with an ear cavum concha of the temperature measurement target person.

This makes it possible to further improve the retentiveness of the ear thermometer in an ear by the holder of the ear thermometer.

A tip portion that is cuttable from the engaging arm may be provided at a tip of the engaging arm.

As a result, since the holder of the ear thermometer accommodates ears of various sizes, this makes it possible to further improve the retentiveness of the ear thermometer.

In a further embodiment, use of the ear thermometer (E) with the holder (F1) for measuring a temperature of an eardrum of an ear of a temperature measurement target person in a non-contact manner, and configured to be worn to an ear hole of the temperature measurement target person is provided

As a result, by the function of the holder, the ear thermometer can be stably held in a state in which an infrared sensor can efficiently receive infrared rays emitted from an eardrum.

With the aspects of the present invention, it is possible to stably hold an ear thermometer in a state where infrared rays emitted from an eardrum can be efficiently received by an infrared sensor.

### BRIEF DESCRIPTION OF THE DRAWINGS

FIG. 1 is a perspective view illustrating an ear thermometer to which a holder according to a first embodiment is attached.
FIG. 2 is a perspective view illustrating the ear thermometer from which the holder according to the first embodiment is removed.
FIG. 3 is a top view of the holder according to the first embodiment.
FIG. 4 is a side view of the holder according to the first embodiment.
FIG. 5 is a front view of the holder according to the first embodiment.
FIG. 6 is a bottom view of the holder according to the first embodiment.
FIG. 7(a) is a right side view illustrating a configuration example of the ear thermometer from which the holder according to the first embodiment is removed, FIG. 7(b) is a bottom view of the same, and FIG. 7(c) is a front view of the same.
FIG. 8 is an explanatory view illustrating a state in which the ear thermometer to which the holder according to the first embodiment is attached is worn to an ear.
FIG. 9(a) is a side view of a holder in an example useful for understanding the invention, FIG. 9(b) is a top view of the same, FIG. 9(c) is a front view of the same, and FIG. 9(d) is a bottom view of the same.
FIG. 10 is an explanatory view illustrating a state in which an ear thermometer to which the holder according to the example useful for understanding the invention is attached is worn to an ear.

### DETAILED DESCRIPTION

### (First Embodiment)

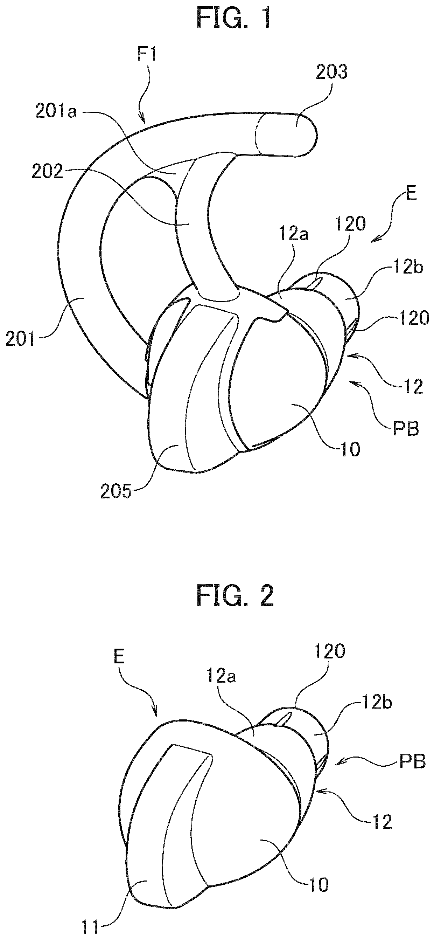

With reference to FIGS. 1 to 8, a holder F1 according to a first embodiment and an ear thermometer E using the holder F1 will be described.

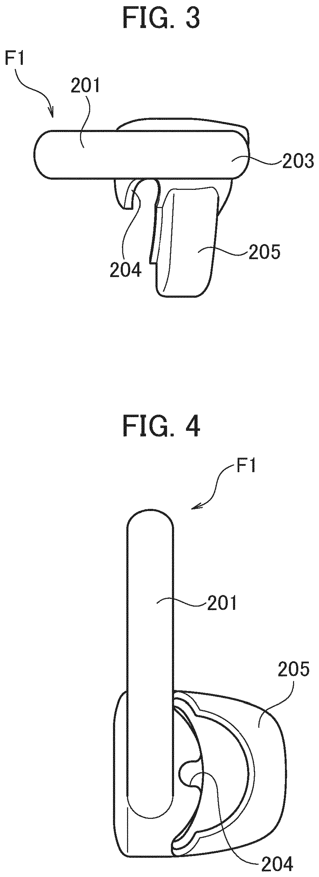

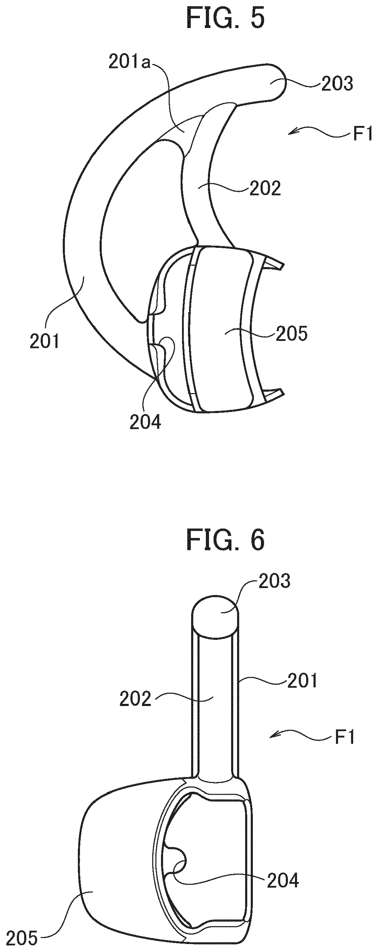

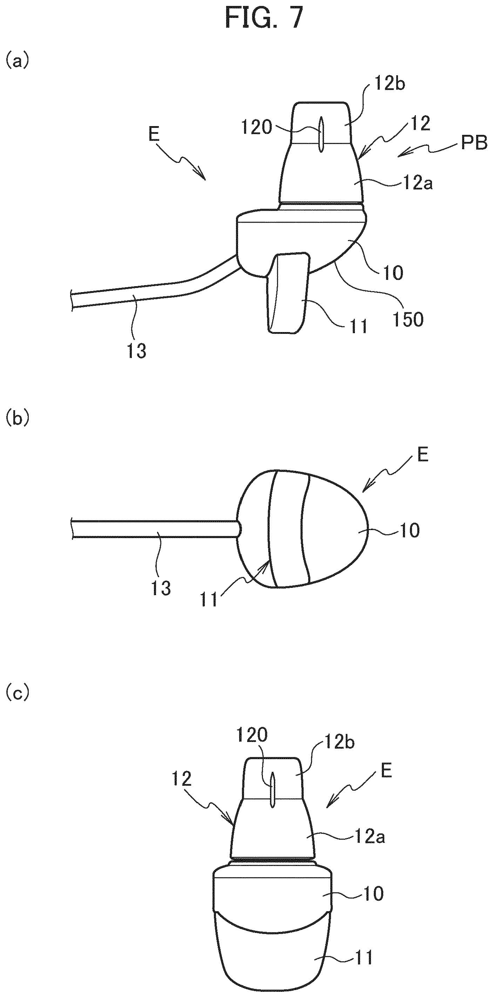

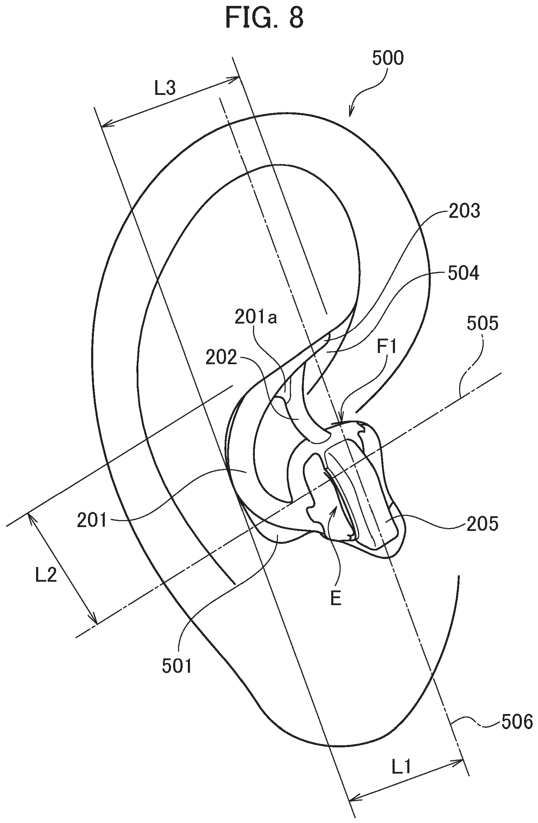

FIG. 1 is a perspective view illustrating the ear thermometer E to which the holder F1 according to the first embodiment is attached. FIG. 2 is a perspective view illustrating the ear thermometer E from which the holder F1 according to the first embodiment is removed. FIG. 3 is a top view of the holder F1. FIG. 4 is a side view of the holder F1. FIG. 5 is a front view of the holder F1. FIG. 6 is a bottom view of the holder F1. FIG. 7(a) is a right side view illustrating a configuration example of the ear thermometer E from which the holder F1 is removed, FIG. 7(b) is a bottom view of the same, and FIG. 7(c) is a front view of the same. FIG. 8 is an explanatory view illustrating a state in which the ear thermometer to which the holder is attached is worn to an ear.

### (Configuration Example of Holder F1)

The holder F1 according to the first embodiment is a holder for the ear thermometer E including an infrared sensor (not illustrated) for measuring a temperature of an eardrum of an ear 500 of a temperature measurement target person such as a patient, a worker, and an athlete in a non-contact manner, and configured to be worn to an ear hole of the temperature measurement target person.

As illustrated in FIGS. 1 to 8, the holder F1 includes a holder body 205 configured to be attached so as to cover a part of a housing 10 of the ear thermometer E, a flexible engaging arm 201 extended from and curved outward the holder body 205 and engageable with a cymba concha 504 (see FIG. 8) of a temperature measurement target person, and a flexible support portion 202 extending from the holder body 205 to join and support the engaging arm 201.

The holder F1 is integrally formed of a soft and flexible material such as silicone rubber.

Further, a flexible joint portion 201a is integrally formed inside an intersection of the engaging arm 201 and the support portion 202.

Further, a part of the engaging arm 201 is formed so as to be engageable with an ear cavum concha 501 of a temperature measurement target person.

Further, as illustrated in FIG. 1 and the like, a cuttable tip portion 203 may be provided at a tip of the engaging arm 201. As a result, it is possible to widely accommodate an ear of various size, which differs from person to person, and to further improve the retentiveness.

In FIG. 3 and the like, a reference numeral 204 is an insertion portion for a cable 13 of the ear thermometer E.

### (Configuration Example of Ear Thermometer E)

As illustrated in FIG. 7, the ear thermometer E includes a probe PB including an infrared sensor (not illustrated in the drawings) for measuring a temperature of an eardrum of the ear 500 of a temperature measurement target person in a non-contact manner, and configured to be worn to an ear hole of the temperature measurement target person.

The probe PB mainly includes a probe body (not illustrated in the drawings) to be inserted into an ear hole of a temperature measurement target person, a housing 10 for supporting the probe body, and an in-ear type earpiece 12 configured to be attached to the probe body and come into contact inside the ear hole of the temperature measurement target person.

The housing 10 and the probe body are molded of ABS resin or the like. The housing 10 and the probe body may be integrally molded.

The infrared sensor is arranged in a recess formed on a tip side of the probe body.

The earpiece 12 includes a base portion 12a that partially exhibits a hollow conical shape and a substantially cylindrical tip portion 12b provided at one end of the base portion 12a and extending in a direction away from the housing 10.

The base portion 12a and the tip portion 12b are integrally formed of a soft and flexible material such as silicon rubber. A reference numeral 11 is a knob portion, and a reference numeral 120 is a hole through which an external sound or the like is passed.

The ear thermometer E having such a configuration can be stably held in an ear hole of a temperature measurement target person even by itself, but by attaching the holder F1 according to the first embodiment, the ear thermometer E can be more stably held in a state where the infrared emitted from an eardrum rays can be efficiently received by the infrared sensor.

### (Example of Wearing)

With reference to FIG. 8, a state in which the ear thermometer E to which the holder F1 according to the first embodiment is attached is worn to an ear will be described.

As illustrated in FIG. 8, the engaging arm 201 of the holder F1 is engaged with the cymba concha 504 of the right ear 500 of the temperature measurement target person. At this time, the engaging arm 201 is urged upward in the figure due to the elasticity of the support portion 202 and the joint portion 201a. As a result, the ear thermometer E is stably held in the right ear 500 of the temperature measurement target person.

Further, a part of the engaging arm 201 comes into contact with the ear cavum concha 501. As a result, the ear thermometer E is stably held by the right ear 500 of the temperature measurement target person.

Here, a size of the holder F1 will be described.

As illustrated in FIG. 8, when the distance from a vertical external ear hole center line 506 to the end of the ear cavum concha 501 is L1, when the distance from a lateral external ear hole center line 505 to the end of the cymba concha 504 is L2, and when the distance from the end of the ear cavum concha 501 to the tip portion 203 of the engaging arm 201 is L3, by determining and combining the sizes of L1 to L3 according to the size of the ear, for example, three sizes of L, M, and S can be prepared.

The size L3 is designed to be slightly longer by providing the tip portion 203, and the tip portion 203 can be cut out according to the size of the ear, so that the size L3 can accommodate ears of various sizes without increasing the number of sizes.

Further, FIG. 8 illustrates a state in which the ear thermometer E to which the holder F1 is attached is worn to the right ear 500 of a temperature measurement target person, but it is also possible to prepare a left-ear wearing model in which an attachment state of the engaging arm 201 of the holder F1 is changed so that the model can be worn to a left ear.

### (Example Useful for Understanding the Invention)

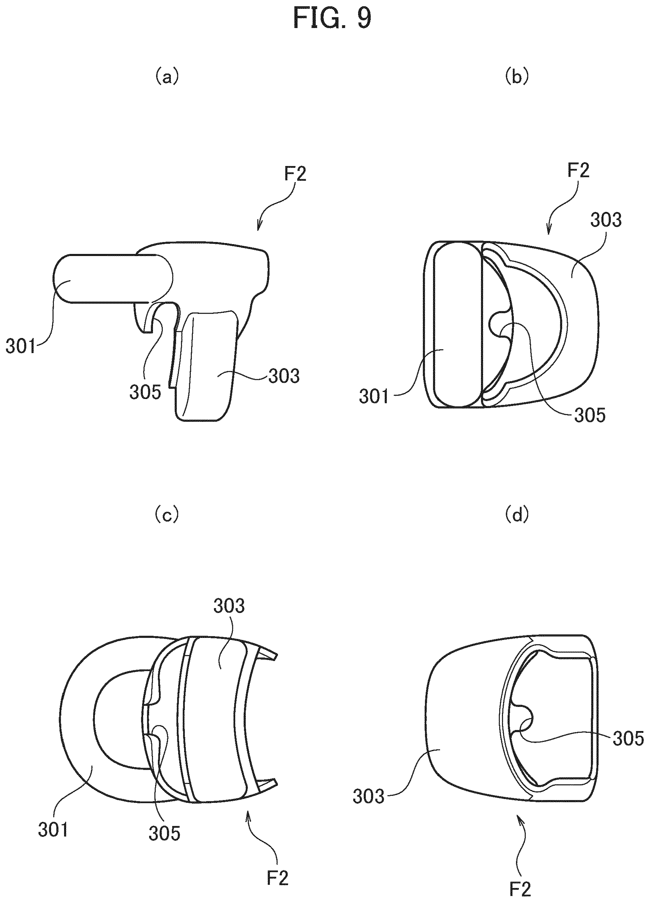

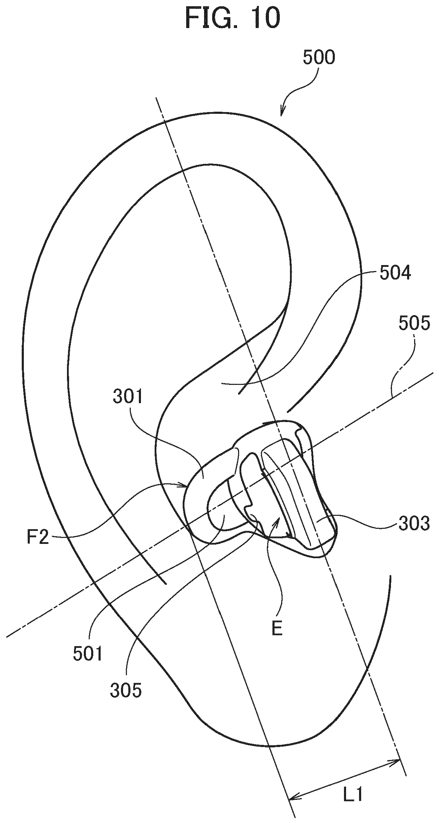

With reference to FIGS. 9 and 10, a holder F2 according to an example useful for understanding the invention and the ear thermometer E using the holder F2 will be described.

FIG. 9(a) is a side view illustrating the holder F2 according to the example useful for understanding the invention, FIG. 9(b) is a top view of the same, FIG. 9(c) is a front view of the same, and FIG. 9(d) is a bottom view of the same. FIG. 10 is an explanatory view illustrating a state in which the ear thermometer E to which the holder F2 is attached is worn to an ear.

A holder according to the example useful for understanding the invention is the holder F2 for an ear thermometer E including an infrared sensor (not illustrated in the drawings) for measuring a temperature of an eardrum of an ear 500 of a temperature measurement target person in a non-contact manner, and configured to be worn to an ear hole of the temperature measurement target person.

The holder F2 includes a holder body 303 configured to attached so as to cover at least a part of the housing of the ear thermometer, and a flexible engaging arm 301 projecting outward from the holder body 303 in an annular manner and configured to be engageable with the ear cavum concha 501 of the temperature measurement target person.

The holder F2 is integrally formed of a soft and flexible material such as silicone rubber.

In FIG. 9, a reference numeral 305 is an insertion portion for the cable 13 of the ear thermometer E.

### (Example of Wearing)

With reference to FIG. 10, a state in which the ear thermometer E to which the holder F2 according to the example useful for understanding the invention is attached is worn to the ear will be described.

As illustrated in FIG. 10, a part of the engaging arm 301 of the holder F2 is engaged with the ear cavum concha 501. As a result, the ear thermometer E is stably held.

Since the holder F2 according to the example useful for understanding the invention can be shared by the left and right ears, convenience can be further improved.

Further, by changing the distance L1 from the vertical external ear hole center line 506 to the end of the ear cavum concha 501, for example, three sizes L, M, and S can be prepared.

## Claims

1. An ear thermometer (E) with a holder (F1), the ear thermometer including an infrared sensor for measuring a temperature of an eardrum of an ear of a temperature measurement target person in a non-contact manner, and configured to be worn to an ear hole of the temperature measurement target person, the holder (F1) comprising:
a holder body (205) configured to be attached to the ear thermometer so as to cover at least a part of the ear thermometer (E),
a flexible engaging arm (201) curved outward and extended from the holder body (205) and configured to be engageable with an ear cymba concha (504) of the temperature measurement target person,
a flexible support portion (202) extending from the holder body (205) to join and support the engaging arm (201), **characterized in that**
a flexible joint portion (201a) is formed inside an intersection of the engaging arm (201) and the support portion (202).

2. The ear thermometer (E) according to claim 1, wherein a part of the engaging arm (201) is formed so as to be engageable with an ear cavum concha (501) of the temperature measurement target person.

3. Use of the ear thermometer (E) with the holder (F1) according to claim 1 or 2 for measuring a temperature of an eardrum of an ear of a temperature measurement target person in a non-contact manner, and configured to be worn to an ear hole of the temperature measurement target person.

## Patentansprüche

1. Ohrthermometer (E) mit einer Halterung (F1), wobei das Ohrthermometer einen Infrarotsensor zur berührungslosen Messung einer Temperatur eines Trommelfells eines Ohrs einer Zielperson der Temperaturmessung einschließt und so konfiguriert ist, dass es in einem Ohrloch der Zielperson der Temperaturmessung getragen werden kann, wobei die Halterung (F1) Folgendes umfasst:
einen Halterungskörper (205), der so konfiguriert ist, dass er am Ohrthermometer so befestigt werden kann, dass er mindestens einen Teil des Ohrthermometers (E) bedeckt,
einen flexiblen Eingriffsarm (201), der nach außen gebogen ist und sich vom Halterungskörper (205) erstreckt und so konfiguriert ist, dass er mit einer Ohrmuschel (504) der Zielperson der Temperaturmessung in Eingriff gebracht werden kann,
einen flexiblen Stützabschnitt (202), der sich vom Halterungskörper (205) erstreckt, um sich an den Eingriffsarm (201) anzufügen und diesen zu stützen, **dadurch gekennzeichnet, dass**
in einem Schnittpunkt des Eingriffsarms (201) und des Stützabschnitts (202) ein flexibler Gelenkabschnitt (201a) ausgebildet ist.

2. Ohrthermometer (E) nach Anspruch 1, wobei ein Teil des Eingriffsarms (201) so geformt ist, dass er mit einer Ohrmuschel (501) der Zielperson der Temperaturmessung in Eingriff gebracht werden kann.

3. Verwendung des Ohrthermometers (E) mit dem Halter (F1) nach Anspruch 1 oder 2 zur berührungslosen Messung einer Temperatur eines Trommelfells eines Ohres einer Zielperson der Temperaturmessung, das so konfiguriert ist, dass es in einem Ohrloch der Zielperson der Temperaturmessung getragen werden kann.

## Revendications

1. Thermomètre d'oreille (E) avec un support (F1), le thermomètre d'oreille incluant un capteur infrarouge pour mesurer une température d'un tympan d'une oreille d'une personne cible de mesure de température d'une manière sans contact, et configuré pour être placé au niveau d'un conduit auditif de la personne cible de mesure de température, le support (F1) comprenant
un corps de support (205) configuré pour être attaché au thermomètre d'oreille de manière à couvrir au moins une partie du thermomètre d'oreille (E),
un bras d'engagement flexible (201) courbé vers l'extérieur et étendu à partir du corps du support (205) et configuré pour pouvoir être engagé avec une cymba conchae d'oreille (504) de la personne cible de mesure de température,
une portion de support flexible (202) s'étendant à partir du corps de support (205) pour joindre et supporter le bras d'engagement (201), **caractérisé en ce que**
une portion de joint flexible (201a) est formée à l'intérieur d'une intersection du bras d'engagement (201) et de la portion de support (202).

2. Thermomètre d'oreille (E) selon la revendication 1, dans lequel une partie du bras d'engagement (201) est formée de manière à pouvoir être engagée avec une cavum conchae d'oreille (501) de la personne cible de mesure de température.

3. Utilisation du thermomètre d'oreille (E) avec le support (F1) selon la revendication 1 ou 2 pour mesurer une température d'un tympan d'une oreille d'une personne cible de mesure de température d'une manière sans contact, et configuré pour être placé au niveau d'un conduit auditif de la personne cible de mesure de température.
